# EUROPEAN PATENT APPLICATION

(11) **EP 4 644 564 A1**
(43) Date of publication of application: **05.11.2025**
(21) Application number: 22969617.4
(22) Date of filing: 28.12.2022
(51) Int. Cl.: C12Q 1/68, C12Q 1/6837, C12Q 1/6874, G01N 31/00

(54) **DETECTION COMPOSITION AND PROTEIN CAPTURE DETECTION METHOD**

(71) Applicant: BGI Shenzhen, Shenzhen, Guangdong 518083 (CN); BGI Shenzhen Co., Ltd, Shenzhen, Guangdong 518083 (CN)
(72) Inventor: HENG, Yang, Shenzhen, Guangdong 518083 (CN); PENG, Jian, Shenzhen, Guangdong 518083 (CN); LIAO, Sha, Shenzhen, Guangdong 518083 (CN); CHEN, Ao, Shenzhen, Guangdong 518083 (CN)
(74) Representative: Gill Jennings & Every LLP
(86) International application number: PCT/CN2022/143032
(87) International publication number: WO 2024/138466

(57) **Abstract**

The present invention relates to the field of biotechnology, and in particular to a detection composition and a protein capture detection method. Provided is a detection composition, comprising a nucleic acid array probe and a protein-nucleic acid conjugate, wherein the nucleic acid array probe comprises a capture sequence, and the capture sequence is used for capturing the protein-nucleic acid conjugate. Simultaneous detection of ultra-high multiplex antibodies can be performed on a same tissue slice. Because spatial reduction of the antibody position depends on sequencing technology, the background staining problem caused by traditional immunohistochemistry and immunofluorescence color developing agents can be solved. In addition, negative control, positive control, and detection of an antibody under test can be carried out on the same tissue slice, a detection result can be determined more accurately, and a false-negative or false-positive result caused by traditional staining technology is eliminated.

## Description

### FIELD

The present disclosure relates to the field of biotechnology, and in particular to a detection composition and a method for capturing and detecting a protein.

### BACKGROUND

Currently, antibody screening at the tissue level for immunohistochemistry or immunofluorescence mainly uses tissue microarrays, where various tissue samples are regularly arranged on a slide and stained with an antibody to see if that antibody can be applied to various types of tissue.

Although tissue microarrays enable high-throughput screening of tissue types, typically it can screen only one type of antibody (in the case of immunohistochemistry) or 1-4 types of antibodies (in the case of immunofluorescence) due to species specificity of the antibodies. Therefore, the throughput for antibody screening using this method is relatively low.

Strictly speaking, each antibody screening process requires negative and positive control stainings performed on the same batch of tissue microarrays, and preferably adjacent sections from the same tissue. However, as the number of antibody types to be screened increases, there may be a significant gap between the sections used for negative and positive controls and those used for each type of antibody staining, thereby resulting in significant differences in cellular composition or antigen type between sections. And this may lead to identification of false negative or false positive outcomes, thereby affecting the accuracy of the screening results.

In tissue microarrays, both immunofluorescence and immunohistochemistry techniques rely on chromogenic reagents or fluorescence to visualize the location of primary antibodies, thereby identifying the positions of their corresponding antigens which bind to the primary antibodies. However, both chromogenic reagents and fluorescence can introduce background staining, which affects the identification of positive results. Additionally, in some tissues, autofluorescence can also affect the identification of positive staining results.

### SUMMARY

In view of this, the present disclosure provides a detection composition and a method for capturing and detecting a protein, particularly a method for high throughput screening antibodies based on nucleic acid arrays.

In order to realize the above objects, the present disclosure provides the following technical solutions.

In a first aspect, the present disclosure provides a detection composition comprising a nucleic acid array probe and a protein-nucleic acid conjugate.

The nucleic acid array probe comprises a capture sequence and the capture sequence is used for capturing the protein-nucleic acid conjugate.

In some specific embodiments of the present disclosure, the nucleic acid array probe further comprises one selected from the group consisting of an excision region, an amplification adaptor B sequence, a spatial localization sequence and a UMI sequence, or a combination thereof;
the excision region is used for releasing an amplification product from a carrier;
the amplification adaptor B sequence is used for amplifying;
the spatial localization sequence is used for spatially localizing the nucleic acid array probe; and
the UMI sequence is used for quantifying and removing a duplicate sequence introduced by amplification.

In some specific embodiments of the present disclosure, the nucleic acid array probe comprises an excision region, an amplification adaptor B sequence, a spatial localization sequence, a UMI sequence and the capture sequence in sequence from the 5' end to the 3' end.

In some specific embodiments of the present disclosure, the protein-nucleic acid conjugate comprises a protein and a nucleic acid sequence.

The nucleic acid sequence comprises a capture complementary sequence, and the capture complementary sequence is complementary to the capture sequence and is used for capturing the protein.

In some specific embodiments of the present disclosure, the nucleic acid sequence further comprises one or two selected from the group consisting of an amplification adaptor A sequence and a protein sequence;
the amplification adaptor A sequence is used for amplifying;
the protein sequence has a unique and definite corresponding relationship to a known nucleic acid sequence and is used for labeling the protein.

In some specific embodiments of the present disclosure, the nucleic acid sequence comprises an amplification adaptor A sequence, a protein sequence and a capture complementary sequence in sequence form 5' end to 3' end.

In some specific embodiments of the present disclosure, the capture complementary sequence has a length not longer than that of the capture sequence.

Based on the above studies, the present disclosure further provides use of the detection composition in detection of a protein.

Further, the present disclosure further provides use of the detection composition in preparing a product for detecting a protein.

In some specific embodiments of the present disclosure, the product includes, but is not limited to, one selected from the group consisting of an agent, a kit, a chip, and a device, or a combination thereof.

In some specific embodiments of the present disclosure, the detection comprises capturing and sequencing,
In some specific embodiments of the present disclosure, the detection comprises high throughput detection on the same tissue section.

In some specific embodiments of the present disclosure, as long as a tissue section can be obtained, any tissue, any fixation method, and any way of obtaining a tissue section are within the protection scope of the present disclosure, and the present disclosure is not limited herein.

In the present disclosure, any of the section and any of the tissue, including but not limited to tissues, as well as cultured cells and organoids, can be used. Lysed cells or tissue lysate can also be used. As long as the substance to be detected can be covered and fixed on the chip, it can theoretically be detected.

In the present disclosure, the above-mentioned any fixation method includes but is not limited to methanol fixation, PFA fixation, formaldehyde fixation, and formalin fixation and paraffin embedding (FFPE). In some cases, different antigen retrieval steps are required according to the different fixation methods.

In the present disclosure, the method of obtaining a tissue section includes but is not limited to obtaining a section by using various microtomes, including freezing microtomes, paraffin slicing machines and vibratomes.

In some specific embodiments of the present disclosure, the high throughput detection comprises detecting one selected from the group consisting of a negative control, a positive control and the protein, or a combination thereof.

In a second aspect, based on the above studies, the present disclosure further provides an agent for capturing or detecting a protein, and the agent comprises the detection composition of the present disclosure and an acceptable auxiliary agent.

In a third aspect, the present disclosure further provides a chip for capturing or detecting a protein, and the detection composition and an acceptable carrier are immobilized on the chip.

In a fourth aspect, the present disclosure further provides a test strip for capturing or detecting a protein, and the detection composition and an acceptable carrier are immobilized on the test strip.

In some specific embodiments of the present disclosure, in the chip, the detection composition provided by the present disclosure can be fixed to the carrier in any manner, and any connection method that can fix the detection composition to the carrier is within the protection scope of the present disclosure, and the present disclosure does not limit this.

For example, the antibody-conjugated nucleic acid can be directly hybridized with the capture sequence on the chip, and the antibody-conjugated nucleic acid and the probe can each be hybridized with a splint oligo and then ligated by a ligase. The antibody-conjugated nucleic acid can also be directly ligated to the probe by other means.

In a fifth aspect, the present disclosure further provides a kit for capturing or detecting a protein, and the kit comprises the detection composition, the agent, the chip or the test strip, and an acceptable excipient, auxiliary agent or carrier.

In some specific embodiments of the present disclosure, the acceptable excipient or auxiliary agent comprises a blocking solution, the blocking solution comprises one selected from the group consisting of serum, salmon sperm DNA, 32 nt PolyA, and WB, or a combination thereof.

In some specific embodiments of the present disclosure, the volume ratio of the serum, the salmon sperm DNA, the 32nt Poly(A) and the WB is 5:5:5:35.

In some specific embodiments of the present disclosure, in the kit, the blocking solution, calculated in a 50 µL system, comprises:

| | |
|---|---|
| serum | 5 µL |
| salmon sperm DNA (10 mg/ml) | 5 µL |
| 32 nt Poly(A) (100 µM) | 5 µL |
| WB | 35 µL |

In a sixth aspect, the present disclosure further provides a device for capturing or detecting a protein, and the device comprises the detection composition, the agent, the chip, the test strip or a component in the kit, and an additional acceptable component.

In a seventh aspect, the present disclosure further provides a method for capturing a protein, and the method comprises combining a sample to be tested with any one selected from the group consisting of:
(I) the detection composition; and/or
(II) the reagent; and/or
(III) the chip; and/or
(IV) the test strip; and/or
(V) a component, test strip or chip in the kit, and performing a reaction to capture the protein.

In an eighth aspect, the present disclosure further provides a method for detecting a protein, and the method comprises combining a sample to be tested with any one selected from the group consisting of:
(I) the detection composition; and/or
(II) the reagent; and/or
(III) the chip; and/or
(IV) the test strip; and/or
(V) a component, test strip or chip in the kit, and
performing a reaction to capture the protein.

In some specific embodiments of the present disclosure, the method comprises the following steps:
step 1: specifically binding the detection composition, the reagent, the chip, the test strip or the protein-nucleic acid conjugate in the kit to a component in the sample to be tested,
step 2: hybridizing the capture sequence of the nucleic acid array probe with the protein-nucleic acid conjugate, extending the nucleic acid array probe to incorporate a sequence complementary to the antibody sequence and a sequence complementary to the amplification adaptor A sequence through nucleic acid amplification, thereby obtaining a nucleic acid with spatial information and antibody information, and
step 3: releasing and recovering the nucleic acid.

In some specific embodiments of the present disclosure, the method further comprises a step of sequencing to determine the type and number of the sample to be tested.

In some specific embodiments of the present disclosure, the nucleic acid comprises one or more selected from the group consisting of the spatial localization sequence, the protein sequence and the UMI sequence.

In some specific embodiments of the present disclosure, the hybridization in step 2 comprises, but is not limited to, permeabilizing a tissue to hybridize the capture sequence of the nucleic acid array probe with the protein-nucleic acid conjugate.

In some specific embodiments of the present disclosure, any method that enables the capture sequence of the nucleic acid array probe to be hybridized with the protein-nucleic acid conjugate is within the protection scope of the present disclosure. For example, the capture of nucleic acids from above a tissue section can be achieved by using a certain device and using a nucleic acid array chip without adding a permeabilizing agent. Devices, components or methods that can achieve the above functions are all within the protection scope of the present disclosure, and the present disclosure is not limited in this aspect.

In some specific embodiments of the present disclosure, the extension in step 2 further comprises extending the protein-nucleic acid conjugate in the case where the capture complementary sequence has a length not longer than that of the capture sequence, to obtain a nucleic acid with spatial information and protein information.

In some specific embodiments of the present disclosure, the capture complementary sequence is designed to have a length equal to or shorter than that of the capture sequence. After extending the probe, the protein-nucleic acid conjugate can also extend to incorporate spatial information and UMI information. This part of the product can be collected using a denaturing agent, and similar results can also be obtained. Any method that can obtain the extension product is within the scope of protection of the present disclosure, and the present disclosure is not limited here.

For example, after the capture probe on the chip extends to incorporate the antibody information and is denatured into a single chain by the denaturing agent, extension can be performed by adding any form of extension primer. In this way, a product with spatial information, antibody information and UMI information can also be obtained.

In some specific embodiments of the present disclosure, the method further comprises a step of detecting whether the protein-nucleic acid conjugate binds specifically to the component in the sample to be tested between step 1 and step 2.

In some specific embodiments of the present disclosure, the method further comprises a step of preparing a tissue section of the sample to be tested before step 1.

In some specific embodiments of the present disclosure, as long as a tissue section can be obtained, any tissue, any fixation method, and any way of obtaining a tissue section are within the protection scope of the present disclosure, and the present disclosure is not limited in this aspect.

In the present disclosure, the any section and any tissue, including but not limited to a tissue, cultured cells or an organoid, can be used. Cell lysis or tissue lysate can also be used. As long as the substance to be detected can cover and be fixed on the chip, it can theoretically be detected.

In the present disclosure, any fixation method includes but is not limited to methanol fixation, PFA fixation, formaldehyde fixation, and formalin fixation and paraffin embedding (FFPE). In some cases, different antigen retrieval steps are required according to different fixation methods.

In the present disclosure, the method of obtaining tissue sections includes but is not limited to obtaining sections by using various microtomes, including a freezing microtome, a paraffin slicing machine and a vibratome.

For the detection composition, the use, the reagent, the chip, the test strip, the kit, the device, or the method, the protein includes, but is not limited to, an antibody. Any protein that can be detected by a specific binding method is within the protection scope of the present disclosure. The present disclosure does not limit the specific binding method herein. As long as the protein can be detected by combining the nucleic acid detection composition provided by the present disclosure, it is a subject matter to be protected in the present disclosure.

The beneficial effects of the present disclosure include, but are not limited to the following effects.

The present disclosure can realize ultra-high-throughput screening of antibody types, and can realize simultaneous screening of at least dozens of antibodies. The present disclosure can perform simultaneous detection of negative control, positive control and multiple antibodies on the same tissue section, which can avoid the false-negative and false-positive results caused by differences of section locations. In the present disclosure, based on the information of an antibody-conjugated nucleic acid captured by a chip, the location of the antibody is determined by a scheme of amplification followed by sequencing, which can avoid the background interference caused by existing imaging schemes, and the interference of the tissue itself in imaging.

### BRIEF DESCRIPTION OF DRAWINGS

For more clearly illustrating embodiments of the examples of the present disclosure or the embodiments in the prior art, drawings used in the examples or the prior art will be briefly described hereinafter.
FIG. 1 shows the structure of the antibody-nucleic acid conjugate.
FIG. 2 shows the structure of the nucleic acid array probe.
FIG. 3 shows the experimental flow of the present disclosure.
FIG. 4 is a schematic diagram of the information of an antibody obtained by the reverse transcription reaction.
FIG. 5 shows the visualization results of 35 antibodies on the same PFA-fixed spleen section.
FIG. 6 shows the comparison results between the technology of the present disclosure and the traditional immunofluorescence technology for the detection of the same type of antibody.

### DETAILED DESCRIPTION

The present disclosure relates to a detection composition and a method for capturing and detecting a protein. Those skilled in the art can realize them by learning from the content herein and appropriately modifying the process parameters. It should be particularly noted that all similar substitutions and modifications are obvious to those skilled in the art, and they are deemed to be included in the present disclosure. The method and the application of the present disclosure have been described through the preferred examples, and it is obvious that the method and application described herein may be changed or appropriately modified and combined to realize and apply the technology of the present disclosure by those skilled in the art without departing from the content, spirit and scope of the present disclosure.

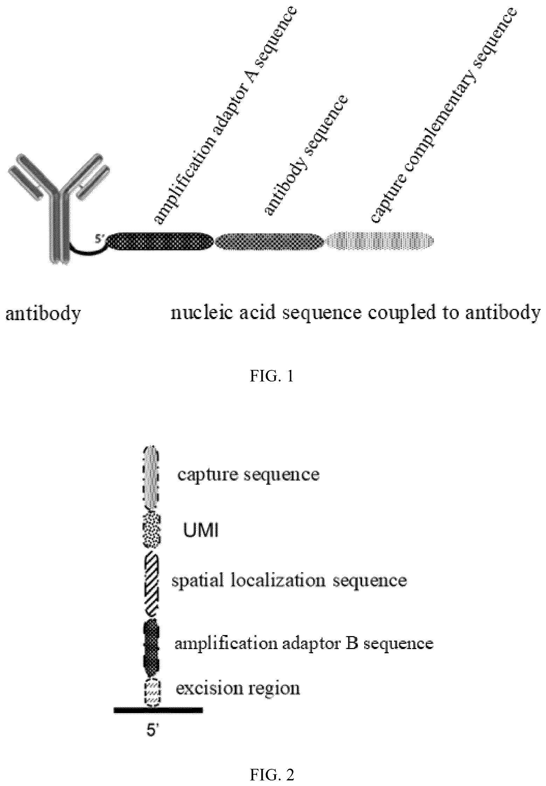

The present disclosure first requires the preparation/purchase of the antibody-nucleic acid conjugate with a nucleic acid coding sequence, and its structure is shown in FIG. 1. The nucleic acid sequence can be divided into three parts. The first part is the amplification adaptor A sequence, which is used for subsequent PCR amplification, the second part is an antibody-specific antibody sequence, each corresponding to a known nucleic acid sequence, which is used for antibody identity, and the third part is the capture complementary sequence complementary to the capture sequence on the chip, which is used for antibody capture.

As shown in FIG. 2, the structure of the nucleic acid array probe used in the present disclosure comprises from 5' to 3': the excision region for subsequent release of the information from the solid phase carrier, the amplification adaptor B sequence for subsequent PCR amplification, the spatial localization sequence for spatial localization of the nucleic acid array, the UMI sequence for quantification and removal of duplicate sequences introduced by subsequent PCR, and the capture sequence for capture of antibody-nucleic acid conjugate.

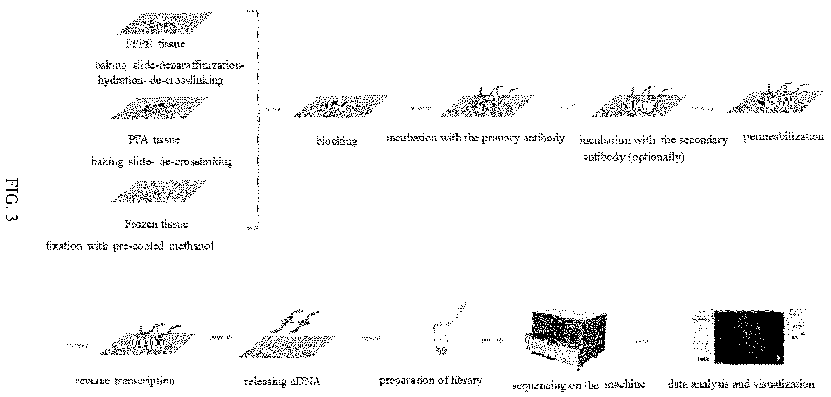

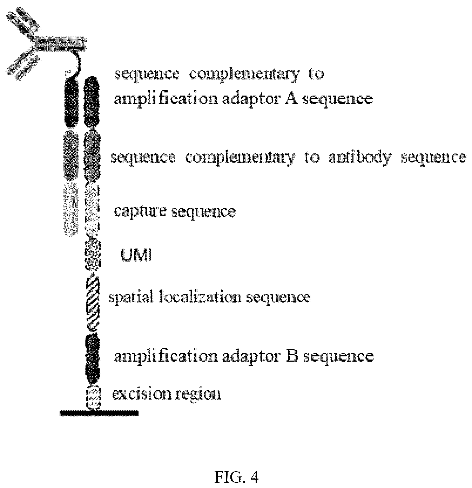

The specific technical route is shown in FIG. 3. After attaching the tissue section to the nucleic acid array, different tissue processing methods are adopted according to different types of tissues. FFPE tissues require processing steps including baking slide to prevent detachment, deparaffinization, hydration, sectioning, and de-crosslinking. PFA tissue requires baking slide to prevent detachment and de-crosslinking. Frozen tissue requires methanol fixation. Then, different types of sample follow the same operating procedures. In a step of blocking, nonspecific binding of the antibody and the conjugated nucleic acid to the tissue and chip is blocked. In a step of incubating the primary antibody, an antibody-nucleic acid conjugate and corresponding negative control antibody-nucleic acid conjugates are added. After a series of washing steps, the antibody-nucleic acid conjugate bound to specific antigen is retained. In order to confirm the successful binding of the antibody-nucleic acid conjugate, secondary antibody incubation can be optionally performed for fluorescent color development and photography. After that, the antibody-conjugated nucleic acid sequence is hybridized with the probe on the chip through tissue permeabilization. Subsequently, through DNA synthesis reactions such as reverse transcription reactions (as shown in FIG 4), probes on the chip are extended to incorporate complementary sequences of antibody sequences and amplification adaptor A sequences, thereby obtaining cDNAs with spatial information and antibody information. After that, these cDNAs are released from the chip, purified and recovered with magnetic beads, prepared for a sequencing library and sequenced on the machine. The spatial localization sequence, antibody sequence, and UMI sequence are sequenced to determine the type and quantity of antibodies bound at a specific spatial location.

According to the present disclosure, ultra-high multiplex antibodies can be simultaneously detected on the same tissue section. Because spatial restoration of the antibody position relies on sequencing technology, the background staining problem caused by traditional immunohistochemistry and immunofluorescence color developing agents can be solved. In addition, the detection of negative control, positive control and an antibody to be tested can be achieved on the same tissue section, the detection result can be determined more accurately, and a false negative or false positive result caused by traditional staining technology can be excluded.

All raw materials and reagents used in the detection composition and the method for capturing or detecting a protein provided by the present disclosure can be purchased from the market. The present disclosure is further described below in conjunction with the examples.

### Example 1 Antibody screening for spleen PFA samples

1. Structure of antibody-nucleic acid conjugate: In this example, the antibody-nucleic acid conjugate was TotalseqA antibody (Biolegend), and 35 types of antibodies recognizing different antigens were applied (see Table 1 for specific types).

Wherein, amplification adaptor A sequences of all antibodies are the following sequence: CCTTGGCACCCGAGAATTCCA (as shown in SEQ ID No. 1), and antibody sequences are shown in Table 1. All their capture complementary sequences are the following sequence: BAAAAAAAAAAAAAAAAAAAAAAAAAAAAAA*A*A (32nt poly(A) sequence, * represents thio modification, as shown in SEQ ID No. 2).

**Table 1: Applied antibodies and corresponding antibody sequences**

| **DNA_I D** | **Description** | **Clone** | **Sequence** | **Number** |
|---|---|---|---|---|
| A0001 | anti-mouse CD4 | RM4-5 | AACAAGACCCTTGAG | SEQ ID No. 3 |
| A0002 | anti-mouse CD8a | 53-6.7 | TACCCGTAATAGCGT | SEQ ID No. 4 |
| A0184 | anti-mouse CD335 (NKp46) | 29A1.4 | CCCTTTCACCTCGAA | SEQ ID No. 5 |
| A0013 | anti-mouse Ly-6C | HK1.4 | AAGTCGTGAGGCATG | SEQ ID No. 6 |
| A0014 | anti-mouse/human CD11b | M1/70 | TGAAGGCTCATTTGT | SEQ ID No. 7 |
| A0015 | anti-mouse Ly-6G | 1A8 | ACATTGACGCAACTA | SEQ ID No. 8 |
| A0073 | anti-mouse/human CD44 | IM7 | TGGCTTCAGGTCCTA | SEQ ID No. 9 |
| A0122 | anti-mouse TER-119/Erythroid Cells | TER-11 9 | GCGCGTTTGTGCTAT | SEQ ID No. 10 |
| A0075 | anti-mouse CD90.2 | 30-H12 | CCGATCAGCCGTTTA | SEQ ID No. 11 |
| A0103 | anti-mouse/human CD45R/B220 | RA3-6B 2 | CCTACACCTCATAAT | SEQ ID No. 12 |
| A0107 | anti-mouse CD21/CD35 (CR2/CR1) | 7 E9 | GGATAATTTCGATCC | SEQ ID No. 13 |
| A0114 | anti-mouse F4/80 | BM8 | TTAACTTCAGCCCGT | SEQ ID No. 14 |
| A0119 | anti-mouse SiglecH | 551 | CCGCACCTACATTAG | SEQ ID No. 15 |
| A0182 | anti-mouse CD3 | 17A2 | GTATGTCCGCTCGAT | SEQ ID No. 16 |
| A0192 | anti-mouse CD20 | SA275 A11 | TCCACTCCCTGTATA | SEQ ID No. 17 |
| A0226 | anti-mouse CD106 | 429 | CGTTCCTACCTACCT | SEQ ID No. 18 |
| A0109 | anti-mouse CD16/32 | 93 | TTCGATGCTGGAGCA | SEQ ID No. 19 |
| A0448 | anti-mouse CD204 | 1F8C33 | AGCTAGACACGTTGT | SEQ ID No. 20 |
| A0417 | anti-mouse CD163 | S15049I | GAGCAAGATTAAGAC | SEQ ID No. 21 |
| A0440 | anti-mouse CD169/Siglec-1 | 3D6.11 2 | ATTGACGACAGTCAT | SEQ ID No. 22 |
| A0441 | anti-mouse CD71 | RI7217 | ACCGACCAGTAGACA | SEQ ID No. 23 |
| A0450 | anti-mouse IgM | RMM-1 | AGCTACGCATTCAAT | SEQ ID No. 24 |
| A0557 | anti-mouse CD38 | 90 | CGTATCCGTCTCCTA | SEQ ID No. 25 |
| A0573 | anti-mouse CD140a | APA5 | GTCATTGCGGTCCTA | SEQ ID No. 26 |
| A0560 | anti-mouse CD68 | FA-11 | CTTTCTTTCACGGGA | SEQ ID No. 27 |
| A0571 | anti-mouse IgD | 11-26c. 2a | TCATATCCGTTGTCC | SEQ ID No. 28 |
| A0825 | anti-mouse CD371 (CLEC12A) | 5D3/CL EC12A | GCGAGAAATCTGCAT | SEQ ID No. 29 |
| A1042 | anti-mouse CD88 (C5aR) | 20/70 | GCAGTCCTACATTGA | SEQ ID No. 30 |
| A0852 | anti-mouse CD226 (DNAM-1) | 10 E5 | ACGCAGTATTTCCGA | SEQ ID No. 31 |
| A0903 | anti-mouse CD40 | 3/23 | ATTTGTATGCTGGAG | SEQ ID No. 32 |
| A0904 | anti-mouse CD31 | 390 | GCTGTAGTATCATGT | SEQ ID No. 33 |
| A0111 | anti-mouse CD5 | 53-7.3 | CAGCTCAGTGTGTTG | SEQ ID No. 34 |
| A0093 | anti-mouse CD19 | 6D5 | ATCAGCCATGTCAGT | SEQ ID No. 35 |
| A0424 | anti-mouse CD14 | Sa14-2 | AACCAACAGTCACGT | SEQ ID No. 36 |
| A0238 | Rat IgG2a, κ Isotype Ctrl Antibody | RTK27 58 | AAGTCAGGTTCGTTT | SEQ ID No.37 |

2. Structure of capture nucleic acid array: in this Example, the probe sequence is CTGCTGACGTACTGAGAGGCATGGCGACCTTATCAGNNNNNNNNNNNNNNNNNNNN NNNNNTTGTCTTCCTAAGACNNNNNNNNNNTTTTTTTTTTTTTTTTTTTTTV (as shown in SEQ ID No. 38). Wherein, the amplification adaptor B sequence is CTGCTGACGTACTGAGAGGCATGGCGACCTTATCAG (as shown in SEQ ID No. 39), the spatial localization sequence is a 25nt random sequence, which was known through sequencing in the course of preparing the nucleic acid array; the UMI is a 10nt random sequence; and the capture sequence is TTTTTTTTTTTTTTTTTTTTTV (as shown in SEQ ID No. 40).

### 3. Preparation of the reagents

Washing Buffer (WB): 1% Triton-X-100 was dissolved in PBS buffer. The single reaction system of the blocking solution is shown in Table 2, and the primary antibody dilution solution was the same as the blocking solution.

**Table 2: preparation of single reaction system of blocking solution**

| component | volume |
|---|---|
| serum | 5 µL |
| salmon sperm DNA (10 mg/ml) | 5 µL |
| 32 nt Poly(A) (100 µM) | 5 µL |
| WB | 35 µL |
| total | 50 µL |

### 4. Specific experimental steps

1) Mouse PFA spleen tissue was sectioned with a freezing microtome at a thickness of 10 µm. After sections were attached to the chip surface, the chip was dried at 37°C for 3 min;
2) Tissue sections were washed 3 times using 50 µL WB with 1 min of incubation per wash;
3) The chip attached to the tissue was transferred to a 24-well plate, added with 400 µL of 4% PFA, and fixed at room temperature for 5 min;
4) The liquid was aspirated and discarded, and the tissue was washed 3 times using 50 µL WB, each time for 1 min;
5) 50ul of blocking solution per chip was added and incubated at room temperature for 30 min;
6) The blocking solution was aspirated and discarded and 35 antibodies were added separately to a primary antibody dilution solution, with 0.5µL for each antibody, the total volume was adjusted to be 50µL, and the resulting primary antibody incubation solution was added to the surface of the tissue section and reacted at room temperature for 40 min;
7) The primary antibody incubation solution was aspirated and discarded, and washing was performed using 50 µL WB 3 times, each time for 1 min;
8) Optionally, the fluorescent secondary antibody was added and incubated at room temperature for 30 min;
9) Optionally, the secondary antibody incubation solution was aspirated and discarded, and washing was performed using 50 µL WB 3 times, each time for 1 min;
10) Optionally, the chip was air dried, sealed by the addition of 4 µl of glycerol after the tissue got dried, and then fluorescence images were collected;
11) Optionally, the coverslide was removed, the chip was placed in 5×SSC solution for 5 min of reaction, and the glycerol was washed off;
12) The chip was taken, washed once with 0.1×SSC, and added with a permeabilizing agent for 8 min of reaction at 37°C;
13) The chip was washed once with RT mix and added with RT mix for 2 h of reaction at 42°C;
14) 400 µL of tissue removal solution was added, incubated at 55°C for 30 min, and the tissue removal solution was removed, and 400 µL of cDNA release reagent was added for 3 h of reaction at 55°C;
15) The released product was collected, the cDNA was purified using 2.0 X magnetic beads, and the PCR amplification was carried out for 18-20 cycles. The forward primer sequence for amplification is 5' phosphorylated CTGCTGACGTACTGAG*A*G*G*C*A*T (as shown in SEQ ID No. 41), where * represents thio modification. The reverse primer sequence is GAGACGTTCTCGACTCAGCAGACCTTGGCACCCGAGAATTCCA (as shown in SEQ ID No. 42).
16) The amplification product was sequenced by using the MGI-2000 sequencer, and the sequencing scheme includes read 1 (25bp spatial localization sequence + 10bp UMI) and read 2 (15bp nucleic acid sequence for antibody);
17) The results of protein detection were obtained through the analysis and visualization system developed by BGI.

### 5. Experimental results

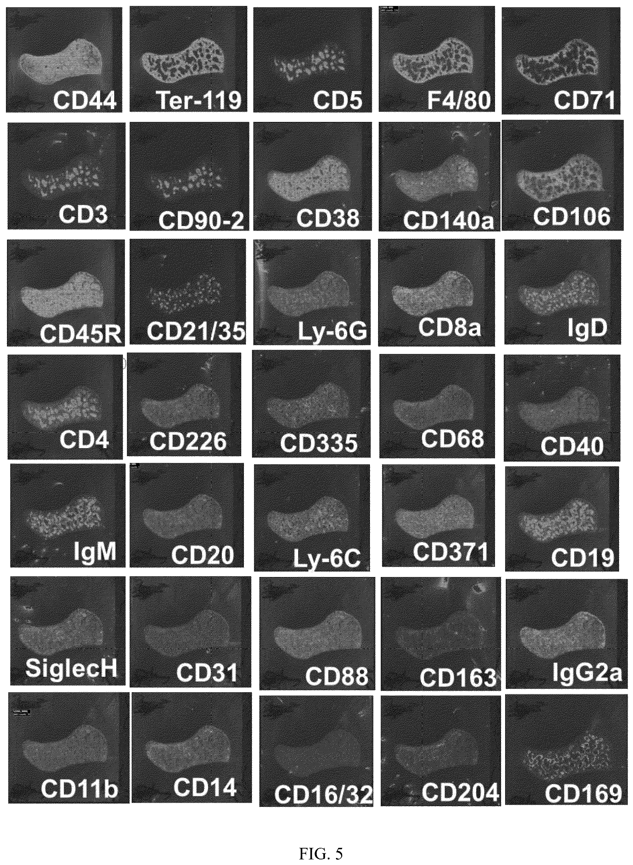

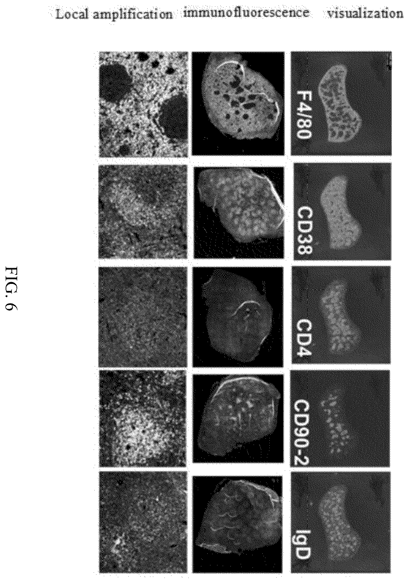

1) A total of 35 types of antibodies were used in this Example, and 35 types of antibodies were detected through sequencing and data analysis. The specific visualization results of each antibody are shown in FIG. 5. Among them, the antibodies of which obvious staining patterns can be obtained were CD44 antibody, TER119 antibody, CD5 antibody, F4/80 antibody, CD71 antibody, CD3 antibody, CD90-2 antibody, CD38 antibody, CD106 antibody, CD45R antibody, CD21/35 antibody, IgD antibody, CD4 antibody, IgM antibody, CD19 antibody and CD169 antibody, a total of 16, proving that these antibodies can be applied to PFA spleen tissue. The CD140a antibody showed a certain pattern, which may be applied to PFA-fixed spleen tissue. There was no obvious difference between the negative control antibody IgG2a and other antibodies, proving that these antibodies were not applicable to PFA-fixed spleen tissue.
2) The 16 antibodies screened above were verified by traditional immunofluorescence technology, and F4/80, CD38, CD4, CD90-2, and IgD antibodies were selected for staining PFA-fixed spleen. The results proved that these antibodies can be applied to PFA-fixed spleen tissue, as shown in FIG. 6. Each type of antibody had a specific staining pattern, and under magnification the staining of single cell membrane structure can be observed. In addition, this technology showed a higher signal-to-noise ratio in the detection of some antibodies, such as CD4 and IgD, compared to traditional immunofluorescence technology.

The detection composition and the method for capturing and detecting a protein provided by the present disclosure are described in detail above. The principle and implementation of the present disclosure are illustrated by using specific embodiments herein. The above descriptions of the examples are only used to facilitate understanding of the method and the core idea of the present disclosure. It should be noted that, several improvements and modifications may be made to the present disclosure by those skilled in the art without departing from the principle of the present disclosure, and the improvements and modifications also fall within the protection scope of the claims.

Sequence Listing

<?xml version="1.0" encoding="UTF-8"?>
<!DOCTYPE ST26SequenceListing SYSTEM "ST26SequenceListing_V1_3.dtd" PUBLIC
"-//WIPO//DTD Sequence Listing 1.3//EN">
<ST26SequenceListing productionDate="2022-12-27" softwareVersion="2.2.0"
softwareName="WIPO Sequence" filieName="OP221685.xml" dtdVersion="V1_3"
nonEnglishFreeTextLanguageCode="zh"
originalFreeTextLanguageCode="zh"><AppplicantFileReference>OP221685</Applicant
FilesReference><ApplicantName languageCode="zh"> BGI SHENZHEN
<ApplicantName><ApplicantNameLatin><BGI
SHENZHEN</ApplicantNameLatin><InventionTitle languageCode="zh"> DETECTION
COMPOSITION AND PROTEIN CAPTURE DETECTION METHOD
</InventionTitle><SequenceTotalQuantity>42</SequenceTotalQuantity>
<SequenceData sequenceIDNumber="1">
<INSDSeq>
<INSDSeq_length>21</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..21</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q2">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ccttggcacccgagaattcca</INSDSeq_sequence>
</INSDSeq>
</SequenceData>
<SequenceData sequenceIDNumber="2">
<INSDSeq>
<INSDSeq_length>31</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..31</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q4">

<INSDQualifier_name>organism</INSDQualifier_name>
<INSDQualifier_value>synthetic construct</INSDQualifier_value>
</INSDQualifier>
</INSDFeature_quals>
</INSDFeature>
<INSDFeature>
<INSDFeature_key>modified_base</INSDFeature_key>
<INSDFeature_location>30..31</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q85">
<INSDQualifier_name>note</INSDQualifier_name>
<NonEnglishQualifier_value>thio-modified a</NonEnglishQualifier_value>
</INSDQualifier>
</INSDFeature_quals>
</INSDFeature>
</INSDSeq_feature-table>
<INSDSeq_sequence>baaaaaaaaaaaaaaaaaaaaaaaaaaaann</INSDSeq_sequence>
</INSDSeq>
</SequenceData>
<SequenceData sequenceIDNumber="3">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q86">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>aacaagacccttgag</INSDSeq_sequence>
</INSDSeq>
</SequenceData>
<SequenceData sequenceIDNumber="4">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>

<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q87">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tacccgtaatagcgt</INSDSeq_sequence>
</INSDSeq>
</SequenceData>
<SequenceData sequenceIDNumber="5">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q88">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ccctttcacctcgaa</INSDSeq_sequence>
</INSDSeq>
</SequenceData>
<SequenceData sequenceIDNumber="6">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q89">
<INSDQualifier_name>organism</INSDQualifier_name>
<INSDQualifier_value>synthetic construct</INSDQualifier_value>

</INSDQualifier>
</INSDFeature_quals>
</INSDFeature>
</INSDSeq_feature-table>
<INSDSeq_sequence>aagtcgtgaggcatg</INSDSeq_sequence>
</INSDSeq>
</SequenceData>
<SequenceData sequenceIDNumber="7">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q90">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tgaaggctcatttgt</INSDSeq_sequence>
</INSDSeq>
</SequenceData>
<SequenceData sequenceIDNumber="8">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q91">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>acattgacgcaacta</INSDSeq_sequence>
</INSDSeq>
</SequenceData>
<SequenceData sequenceIDNumber="9">

<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q92">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tggcttcaggtccta</INSDSeq_sequence>
</INSDSeq>
</SequenceData>
<SequenceData sequenceIDNumber="10">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q93">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gcgcgtttgtgctat</INSDSeq_sequence>
</INSDSeq>
</SequenceData>
<SequenceData sequenceIDNumber="11">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>

<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q94">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ccgatcagccgttta</INSDSeq_sequence>
</INSDSeq>
</SequenceData>
<SequenceData sequenceIDNumber="12">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q95">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>cctacacctcataat</INSDSeq_sequence>
</INSDSeq>
</SequenceData>
<SequenceData sequenceIDNumber="13">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q96">
<INSDQualifier_name>organism</INSDQualifier_name>
<INSDQualifier_value>synthetic construct</INSDQualifier_value>

</INSDQualifier>
</INSDFeature_quals>
</INSDFeature>
</INSDSeq_feature-table>
<INSDSeq_sequence>ggataatttcgatcc</INSDSeq_sequence>
</INSDSeq>
</SequenceData>
<SequenceData sequenceIDNumber="14">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q97">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ttaacttcagcccgt</INSDSeq_sequence>
</INSDSeq>
</SequenceData>
<SequenceData sequenceIDNumber="15">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q98">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ccgcacctacattag</INSDSeq_sequence>
</INSDSeq>
</SequenceData>
<SequenceData sequenceIDNumber="16">

<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q99">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gtatgtccgctcgat</INSDSeq_sequence>
</INSDSeq>
</SequenceData>
<SequenceData sequenceIDNumber="17">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q100">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tccactccctgtata</INSDSeq_sequence>
</INSDSeq>
</SequenceData>
<SequenceData sequenceIDNumber="18">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>

<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q101">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>cgttcctacctacct</INSDSeq_sequence>
</INSDSeq>
</SequenceData>
<SequenceData sequenceIDNumber="19">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q102">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ttcgatgctggagca</INSDSeq_sequence>
</INSDSeq>
</SequenceData>
<SequenceData sequenceIDNumber="20">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q103">
<INSDQualifier_name>organism</INSDQualifier_name>
<INSDQualifier_value>synthetic construct</INSDQualifier_value>

</INSDQualifier>
</INSDFeature_quals>
</INSDFeature>
</INSDSeq_feature-table>
<INSDSeq_sequence>agctagacacgttgt</INSDSeq_sequence>
</INSDSeq>
</SequenceData>
<SequenceData sequenceIDNumber="21">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q104">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gagcaagattaagac</INSDSeq_sequence>
</INSDSeq>
</SequenceData>
<SequenceData sequenceIDNumber="22">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q105">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>attgacgacagtcat</INSDSeq_sequence>
</INSDSeq>
</SequenceData>
<SequenceData sequenceIDNumber="23">

<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q106">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>accgaccagtagaca</INSDSeq_sequence>
</INSDSeq>
</SequenceData>
<SequenceData sequenceIDNumber="24">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q107">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>agctacgcattcaat</INSDSeq_sequence>
</INSDSeq>
</SequenceData>
<SequenceData sequenceIDNumber="25">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>

<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q108">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>cgtatccgtctccta</INSDSeq_sequence>
</INSDSeq>
</SequenceData>
<SequenceData sequenceIDNumber="26">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q109">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gtcattgcggtccta</INSDSeq_sequence>
</INSDSeq>
</SequenceData>
<SequenceData sequenceIDNumber="27">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q110">
<INSDQualifier_name>organism</INSDQualifier_name>
<INSDQualifier_value>synthetic construct</INSDQualifier_value>

</INSDQualifier>
</INSDFeature_quals>
</INSDFeature>
</INSDSeq_feature-table>
<INSDSeq_sequence>ctttctttcacggga</INSDSeq_sequence>
</INSDSeq>
</SequenceData>
<SequenceData sequenceIDNumber="28">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q111">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tcatatccgttgtcc</INSDSeq_sequence>
</INSDSeq>
</SequenceData>
<SequenceData sequenceIDNumber="29">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q112">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gcgagaaatctgcat</INSDSeq_sequence>
</INSDSeq>
</SequenceData>
<SequenceData sequenceIDNumber="30">

<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q113">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gcagtcctacattga</INSDSeq_sequence>
</INSDSeq>
</SequenceData>
<SequenceData sequenceIDNumber="31">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q114">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>acgcagtatttccga</INSDSeq_sequence>
</INSDSeq>
</SequenceData>
<SequenceData sequenceIDNumber="32">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>

<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q115">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>atttgtatgctggag</INSDSeq_sequence>
</INSDSeq>
</SequenceData>
<SequenceData sequenceIDNumber="33">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q116">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gctgtagtatcatgt</INSDSeq_sequence>
</INSDSeq>
</SequenceData>
<SequenceData sequenceIDNumber="34">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q117">
<INSDQualifier_name>organism</INSDQualifier_name>
<INSDQualifier_value>synthetic construct</INSDQualifier_value>

</INSDQualifier>
</INSDFeature_quals>
</INSDFeature>
</INSDSeq_feature-table>
<INSDSeq_sequence>cagctcagtgtgttg</INSDSeq_sequence>
</INSDSeq>
</SequenceData>
<SequenceData sequenceIDNumber="35">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q118">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>atcagccatgtcagt</INSDSeq_sequence>
</INSDSeq>
</SequenceData>
<SequenceData sequenceIDNumber="36">
<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q119">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>aaccaacagtcacgt</INSDSeq_sequence>
</INSDSeq>
</SequenceData>
<SequenceData sequenceIDNumber="37">

<INSDSeq>
<INSDSeq_length>15</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..15</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q120">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>aagtcaggttcgttt</INSDSeq_sequence>
</INSDSeq>
</SequenceData>
<SequenceData sequenceIDNumber="38">
<INSDSeq>
<INSDSeq_length>108</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..108</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q121">
<INSDQualifier_name>organism</INSDQualifier_name>
<INSDQualifier_value>synthetic construct</INSDQualifier_value>
</INSDQualifier>
</INSDFeature_quals>
</INSDFeature>
<INSDFeature>
<INSDFeature_key>modified_base</INSDFeature_key>
<INSDFeature_location>37..61</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q122">
<INSDQualifier_name>note</INSDQualifier_name>
<NonEnglishQualifier_value>n=a,t,c or g</NonEnglishQualifier_value>
</INSDQualifier>
</INSDFeature_quals>

</INSDFeature>
<INSDFeature>
<INSDFeature_key>modified_base</INSDFeature_key>
<INSDFeature_location>77..86</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q123">
<INSDQualifier_name>note</INSDQualifier_name>
<NonEnglishQualifier_value>n=a,t,c or g</NonEnglishQualifier_value>
</INSDQualifier>
</INSDFeature_quals>
</INSDFeature>
</INSDSeq_feature-table>
<INSDSeq_sequence>ctgctgacgtactgagaggcatggcgaccttatcagnnnnnnnnnnnnnnnnnnnn
nttgtcttcctaagacnnnnnnnnnnttttttttttttttttttv</INSDSeq_sequence>
</INSDSeq>
</SequenceData>
<SequenceData sequenceIDNumber="39">
<INSDSeq>
<INSDSeq_length>36</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..36</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q124">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>ctgctgacgtactgagaggcatggcgaccttatcag</INSDSeq_sequence>
</INSDSeq>
</SequenceData>
<SequenceData sequenceIDNumber="40">
<INSDSeq>
<INSDSeq_length>22</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>

<INSDQualifier_value>other DNA</INSDQualifier_value>
</INSDQualifier>
<INSDQualifier id="q125">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>tttttttttttttttttttttv</INSDSeq_sequence>
</INSDSeq>
</SequenceData>
<SequenceData sequenceIDNumber="41">
<INSDSeq>
<INSDSeq_length>22</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..22</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q126">
<INSDQualifier_name>organism</INSDQualifier_name>
<INSDQualifier_value>synthetic construct</INSDQualifier_value>
</INSDQualifier>
</INSDFeature_quals>
</INSDFeature>
<INSDFeature>
<INSDFeature_key>modified_base</INSDFeature_key>
<INSDFeature_location>1</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q127">
<INSDQualifier_name>note</INSDQualifier_name>
<NonEnglishQualifier_value>n is phosphorylated c</NonEnglishQualifier_value>
</INSDQualifier>
</INSDFeature_quals>
</INSDFeature>
<INSDFeature>
<INSDFeature_key>modified_base</INSDFeature_key>
<INSDFeature_location>17</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q128">
<INSDQualifier_name>note</INSDQualifier_name>

<NonEnglishQualifier_value>n is thio-modified a</NonEnglishQualifier_value>
</INSDQualifier>
</INSDFeature_quals>
</INSDFeature>
<INSDFeature>
<INSDFeature_key>modified_base</INSDFeature_key>
<INSDFeature_location>18..19</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q129">
<INSDQualifier_name>note</INSDQualifier_name>
<NonEnglishQualifier_value>n is thio-modified g</NonEnglishQualifier_value>
</INSDQualifier>
</INSDFeature_quals>
</INSDFeature>
<INSDFeature>
<INSDFeature_key>modified_base</INSDFeature_key>
<INSDFeature_location>20</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q130">
<INSDQualifier_name>note</INSDQualifier_name>
<NonEnglishQualifier_value>n is thio-modified c</NonEnglishQualifier_value>
</INSDQualifier>
</INSDFeature_quals>
</INSDFeature>
<INSDFeature>
<INSDFeature_key>modified_base</INSDFeature_key>
<INSDFeature_location>21</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q131">
<INSDQualifier_name>note</INSDQualifier_name>
<NonEnglishQualifier_value>n is thio-modified a</NonEnglishQualifier_value>
</INSDQualifier>
</INSDFeature_quals>
</INSDFeature>
<INSDFeature>
<INSDFeature_key>modified_base</INSDFeature_key>
<INSDFeature_location>22</INSDFeature_location>
<INSDFeature_quals>
<INSDQualifier>
<INSDQualifier_name>mod_base</INSDQualifier_name>
<INSDQualifier_value>OTHER</INSDQualifier_value>
</INSDQualifier>
<INSDQualifier id="q132">
<INSDQualifier_name>note</INSDQualifier_name>

<NonEnglishQualifier_value>n 为硫代修饰的 t</NonEnglishQualifier_value>
</INSDQualifier>
</INSDFeature_quals>
</INSDFeature>
</INSDSeq_feature-table>
<INSDSeq_sequence>ctgctgacgtactgagnnnnnn</INSDSeq_sequence>
</INSDSeq>
</SequenceData>
<SequenceData sequenceIDNumber="42">
<INSDSeq>
<INSDSeq_length>43</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..43</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q133">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>gagacgttctcgactcagcagaccttggcacccgagaattcca</INSDSeq_sequenc
e>
</INSDSeq>
</SequenceData>
</ST26SequenceListing>

## Claims

1. A detection composition comprising a nucleic acid array probe and a protein-nucleic acid conjugate,
wherein the nucleic acid array probe comprises a capture sequence and the capture sequence is used for capturing the protein-nucleic acid conjugate.

2. The detection composition according to claim 1, wherein the nucleic acid array probe further comprises one selected from the group consisting of an excision region, an amplification adaptor B sequence, a spatial localization sequence and a UMI sequence, or a combination thereof;
the excision region is used for releasing an amplification product from a carrier,
the amplification adaptor B sequence is used for amplifying,
the spatial localization sequence is used for spatially localizing the nucleic acid array, and
the UMI sequence is used for quantifying and removing a duplicate sequence introduced by amplification.

3. The detection composition according to claim 1 or 2, wherein the nucleic acid array probe comprises an excision region, an amplification adaptor B sequence, a spatial localization sequence, a UMI sequence and the capture sequence in sequence from 5' end to 3' end.

4. The detection composition according to claim 3, wherein the protein-nucleic acid conjugate comprises a protein and a nucleic acid sequence,
wherein the nucleic acid sequence comprises a capture complementary sequence, and the capture complementary sequence is complementary to the capture sequence and is used for capturing the protein.

5. The detection composition according to claim 4, wherein the nucleic acid sequence further comprises one or two selected from the group consisting of an amplification adaptor A sequence and a protein sequence,
the amplification adaptor A sequence is used for amplifying, and
the protein sequence has a unique and definite corresponding relationship to a known nucleic acid sequence and is used for labeling the protein.

6. The detection composition according to any one of claims 1 to 5, wherein the nucleic acid sequence comprises an amplification adaptor A sequence, a protein sequence and a capture complementary sequence in sequence from5' end to 3' end,
preferably, the capture complementary sequence has a length not longer than that of the capture sequence.

7. Use of the detection composition according to any one of claims 1 to 6 in detection of a protein.

8. Use of the detection composition according to any one of claims 1 to 6 in preparing a product for detection of a protein.

9. The use according to claim 7 or 8, wherein the product includes, but is not limited to, one selected from the group consisting of an agent, a kit, a chip, and a device, or a combination thereof.

10. The use according to any one of claims 7 to 9, wherein the detection comprises capturing and sequencing,
preferably, the detection comprises high throughput detection on a same tissue section, or
preferably, the high throughput detection comprises detecting one selected from the group consisting of a negative control, a positive control and the protein, or a combination thereof.

11. A reagent, comprising the detection composition according to any one of claims 1 to 6 and an acceptable auxiliary agent.

12. A chip, on which the detection composition according to any one of claims 1 to 6 and an acceptable carrier are immobilized.

13. A test strip, on which the detection composition according to any one of claims 1 to 6 and an acceptable carrier are immobilized.

14. A kit comprising the detection composition according to any one of claims 1 to 6, the reagent according to claim 11, the chip according to claim 12 or the test strip according to claim 13, and an acceptable excipient, auxiliary agent or carrier.

15. The kit according to claim 14, wherein the acceptable excipient or auxiliary agent comprises a blocking solution, and the blocking solution comprises one selected from the group consisting of serum, salmon sperm DNA, 32 nt PolyA, and WB, or a combination thereof.

16. A device comprising the detection composition according to any one of claims 1 to 6, the reagent according to claim 11, the chip according to claim 12, the test strip according to claim 13 or a component in the kit according to claim 14 or 15, and an additional acceptable component.

17. A method for capturing a protein, comprising combining a sample to be tested with any one selected from the group consisting of:
(I) the detection composition according to any one of claims 1 to 6, and/or
(II) the reagent according to claim 11, and/or
(III) the chip according to claim 12, and/or
(IV) the test strip according to claim 13, and/or
(V) a component, test strip or chip in the kit according to claim 14 or 15, and
performing a reaction to capture the protein.

18. A method for detecting a protein, comprising combining a sample to be tested with any one selected from the group consisting of
(I) the detection composition according to any one of claims 1 to 6, and/or
(II) the reagent according to claim 11, and/or
(III) the chip according to claim 12, and/or
(IV) the test strip according to claim 13, and/or
(V) a component, test strip or chip in the kit according to claim 14 or 15, and
performing a reaction to capture the protein.

19. The method according to claim 17 or 18, wherein the method comprises:
step 1: specifically binding the detection composition according to any one of claims 1 to 6, the reagent according to claim 11, the chip according to claim 12, the test strip according to claim 13 or the protein-nucleic acid conjugate in the kit according to claim 14 or 15 to a component in the sample to be tested,
step 2: hybridizing the capture sequence of the nucleic acid array probe with the protein-nucleic acid conjugate, extending the nucleic acid array probe to incorporate a sequence complementary to the antibody sequence and a sequence complementary to the amplification adaptor A sequence through nucleic acid amplification, thereby obtaining a nucleic acid with spatial information and antibody information, and
step 3: releasing and recovering the nucleic acid.

20. The method according to claim 19, wherein the method further comprises a step of sequencing to determine the type and number of the sample to be tested,
preferably, the nucleic acid comprises one selected from the group consisting of the spatial localization sequence, the protein sequence and the UMI sequence, or a combination thereof;
preferably, the hybridization in step 2 comprises, but is not limited to, permeabilizing a tissue to hybridize the capture sequence of the nucleic acid array probe with the protein-nucleic acid conjugate;
preferably, the extension in step 2 further comprises extending the protein-nucleic acid conjugate in the case where the capture complementary sequence has a length not longer than that of the capture sequence to obtain a nucleic acid with spatial information and protein information;
preferably, the method further comprises a step of detecting whether the protein-nucleic acid conjugate binds specifically to a component in the sample to be tested between step 1 and step 2; or
preferably, the method further comprises a step of preparing a tissue section of the sample to be tested before step 1.

21. The detection composition according to any one of claims 1 to 6, the use according to any one of claims 7 to 10, the reagent according to claim 11, the chip according to claim 12, the test strip according to claim 13, the kit according to claim 14 or 15, the device according to claim 16 or the method according to any one of claims 17 to 20, wherein the protein includes, but is not limited to, an antibody.
